# EUROPÄISCHE PATENTSCHRIFT

(11) **EP 0 668 074 B1**
(45) Veröffentlichungstag und Bekanntmachung des Hinweises auf die Patenterteilung: **09.05.2001**
(21) Anmeldenummer: 95810045.5
(22) Anmeldetag: 25.01.1995
(51) Int. Cl.: A61K 9/70, A61K 31/37

(54) **Transdermales therapeutisches System**
Transdermal therapeutic system
Système thérapeutique transdermique

(30) Priorität: 18.02.1994 CH 49594
(43) Veröffentlichungstag der Anmeldung: 23.08.1995
(73) Patentinhaber: Drossapharm AG, CH-4059 Basel (CH)
(72) Erfinder: Lutz, Jürg, CH-4102 Binningen (CH); Cierpka, Henning F., Dr., CH-4153 Reinach (CH)
(74) Vertreter: Eder, Carl E.

(56) Entgegenhaltungen:
- EP-A- 0 127 468
- EP-A- 0 391 172
- WO-A-85/03865
- PATENT ABSTRACTS OF JAPAN vol. 6, no. 239 (C-137) & JP-A-57 142 913 (NITTO DENKI KOGYO KK) 3. September 1982
- DATABASE WPI Week 8241, Derwent Publications Ltd., London, GB; AN 82-86701E & JP-A-57 142 913 (NITTO ELECTRIC IND KK) 3. September 1982
- ARZNEIM.-FORSCH./DRUG RES., Bd.38(II), Nr.12, Dezember 1988, AULENDORF (DE) Seiten 1774 - 1777 W.A. RITSCHEL ET AL. 'use of sorption promotors to increase systemic absorption of coumarin from transdermal drug delivery systems'

## Beschreibung

Die Erfindung betrifft ein transdermales therapeutisches System gemäss dem Oberbegriff des Anspruchs 1.

Es ist bekannt, dass der pharmazeutische Wirkstoff 5,6-Benzo-alpha-pyron, nachfolgend auch Benzopyron genannt, antiphlogistische und analgetische, sowie auch antiödematöse und lymphokinetische Eigenschaften besitzt. Aufgrund dieser pharmakologischen Eigenschaften werden Benzopyron enthaltende Arzneimittel zur Behandlung verschiedener, vor allem venöser Gefässerkrankungen sowie auch zur Behandlung proteinreicher Oedeme, besonders zur Therapie proteinreicher Lymphödeme, eingesetzt. Zu den mit Benzopyron-Präparaten behandelbaren Gefässerkrankungen gehören auch die chronisch-venöse Insuffizienz und die Phlebitis. In den letzten Jahren wurde Benzopyron auch zur Behandlung von Krebserkrankungen herangezogen.

Bei vielen Arzneimitteln ist es erwünscht, gleichmässige und langanhaltende, therapeutisch ausreichende Blutspiegel zu erzielen. Dies kann z.B. auf oralem Wege durch eine sog. slow release-Formulierung oder auf transdermalem Wege durch eine Salbe oder dergleichen erreicht werden. Es ist nun bekannt, Benzopyron perkutan, in Form einer Salbe oder eines Gels, zu applizieren. Diese Applikationsweise über die Haut hat den Vorteil, eine "first pass"-Metabolisierung des Wirkstoffes in der Leber weitgehend zu vermeiden. Diese perkutane Applikation von Benzopyron in Form einer Salbe oder einer Creme, wie sie u.a. auch in der US-P 5 096 887 offenbart wird, erlaubt zwar im Einzelfall und kurzfristig die therapeutische Anwendung auf verhältnismässig grossen Hautflächen. Jedoch ist bei einer derartigen Applikation, insbesondere auch angesichts der bekannten, nachfolgend näher beschriebenen individuell sehr unterschiedlichen Hautpenetration (Resorptionsfähigkeit) keine genaue Angabe über die jeweils absorbierte Dosis möglich. Wegen der sich daraus ergebenden Möglichkeit einer Ueberdosierung und der damit verbundenen potentiellen Nebenwirkungen eignen sich solche perkutane Applikationsformen unter dem Aspekt der Arzneimittelsicherheit nicht für eine Daueranwendung. Gerade die Daueranwendung ist aber für eine erfolgreiche Therapie chronischer Erkrankungen der vorgenannten Art mit Benzopyron unbedingt erforderlich.

Ein weiterer Nachteil der vorgenannten perkutanen Präparate besteht darin, dass diese das Benzopyron mit einer exponentiell fallenden Rate (Pharmakokinetik 1. oder 2. Ordnung) freisetzen. Um dennoch eine einigermassen konstante Aufnahme eines Wirkstoffs durch die Haut zu erreichen, muss die Applikation entsprechend sehr häufig wiederholt werden, was für die Patienten lästig und wegen der oft mangelhaften Disziplin der Patienten für den Therapieerfolg nachteilig ist.

Ueber die Penetration von Benzopyron durch die menschliche Haut war bisher wenig bekannt.

Bei der Ratte wurde die Hautpenetration von Benzopyron in der Publikation von Ritschel und Hussain(Meth. Find. Exptl. Clin. Pharmacol. 10,165-169 (1988)) beschrieben. Die Autoren haben bei drei verschiedenen Ratten je 1 g einer 5%igen Benzopyronsalbe auf eine Hautfläche von jeweils 30 cm² aufgetragen. Aus dieser Publikation (dort Tab.3) geht hervor, dass bei dieser Versuchsreihe eine sehr hohe interindividuelle Streuung der Hautpermeabilität von Benzopyron auftrat. So betrug nach 12 Stunden der nicht resorbierte Anteil von Benzopyron bei den drei Ratten 1,95 bzw. 13,24 bzw. 42,8 Gewichtsprozent der ursprünglich aufgetragenen Benzopyrondosis. Gemäss Abb. 3 und 5 dieser Publikation erfolgt die individuelle Absorption durch die Rattenhaut nicht linear, d.h., nicht nach einer Kinetik 0.Ordnung ("there is a deviation from the zero-order absorption profile"; zitiert von S.167/168).

Diese Ergebnisse lassen darauf schliessen, dass bei Applikation von Benzopyron als Salbe sehr unterschiedliche Resorptionsraten zu erwarten sind und dass diese Applikationsform schon aus diesem Grunde nicht für die Langzeittherapie geeignet ist.

In diesem Zusammenhang sei aber darauf hingewiesen, dass sich auch positivere als die vorgenannten Ergebnisse von Penetrationsversuchen bei Ratten nicht ohne weiteres auf den Menschen übertragen lassen: Bei der vorstehend beschriebenen Applikation der Salbe auf 30 cm² der Rattenhaut beträgt das Verhältnis von Applikationsfläche zu Körpergewicht 120 cm²/kg, wenn man das normale Gewicht einer Ratte mit 250 g annimmt. Uebertragen auf einen 75 kg schweren Erwachsenen müsste (gleiche Penetrationseigenschaften der Haut vorausgesetzt), die entsprechende Applikationsfläche 9000 cm² betragen, also ca. 50 % der gesamtem Hautoberfläche umfassen. Auch aus diesem Grund sind direkte Vergleiche der Hautpenetration zwischen Ratten und Menschen nur bedingt zulässig. Abgesehen davon ist die menschliche Haut wesentlich schlechter penetrierbar als die Rattenhaut: Beispielsweise lässt die Rattenhaut nach einer anderen Publikation der gleichen Autoren (Ritschel und Hussain, Arzneimittelforschg.38, 1630-1632, 1988) den Wirkstoff Griseofulvin 14 x besser penetrieren als die zum Vergleich herangezogene menschliche Haut, und das Insektizid Cypermethrin penetriert die Rattenhaut sogar 20 x besser als die menschliche Haut (Scott und Ramsay, J. Invest. Dermatol.89, 142-146, 1987).

Transkutan applizierbare Wirkstoffe lassen sich nicht nur in Form von Salben etc. sondern im Prinzip auch in Form von sogenannten transdermalen therapeutischen Systemen (TDS) verabreichen. Ein TDS ist bekanntlich eine Heftpflaster-artige Vorrichtung mit einem Wirkstoffreservoir, das den Wirkstoff in Form einer galenischen Formulierung enthält und diesen über eine längere Zeit an die Haut abgibt.

Da die menschliche Haut grundsätzlich eine sehr geringe Penetrierbarkeit besitzt, lassen sich nach Ansicht eines Fachmannes bei weitem nicht alle Wirkstoffe mit einem TDS verabreichen, zumal dessen Fläche und die über diese Fläche auf und durch die Haut applizierbare Menge an Wirkstoff sehr begrenzt ist: Beispielsweise eignen sich gemäss EP-A-0 391 172 (dort Seite 3, Zeile 34) Wirkstoffe für die Anwendung in TDS nur, wenn ihre Tagesdosis unter 50 mg liegt.

Da die therapeutisch angewendete und zu ca. 100% resorbierte Dosis von Benzopyron bei oraler Verabreichung bei 100-400 mg/Tag liegt, und in publizierten klinischen Studien versuchsweise per os schon bis zu 7 g pro Tag verabreicht wurden, erschien es wenig aussichtsreich, den Weg über die Entwicklung eines transdermalen therapeutischen Systems zu beschreiten, weil sich diese Systeme, wie vorstehend angeführt, grundsätzlich nur für die Applikation kleiner bis mittlerer Mengen gut penetrierender Wirkstoffe eignen und weil die Applikationsfläche nicht beliebig gross gestaltet werden kann.

Bei den heute üblichen TDS mit Matrixsystemen wird der Wirkstoff in Form von Inseln (EP-A-0 391 172) oder in anderer Form in einer in Wasser unlöslichen Polymermatrix verteilt. Allerdings kann in diesen Fällen vom Prinzip her nur eine sehr begrenzte Menge Wirkstoff von dieser Polymermatrix aufgenommen werden.

Die vorgenannten Ausführungen machen deutlich, wieso auf dem Arzneimittelmarkt für humantherapeutische Zwecke mehrheitlich orale Präparate und nur relativ sehr wenige transdermale therapeutische Systeme erhältlich sind.

Der Erfindung lag nun die Aufgabe zugrunde, ein Arzneimittel zur transdermalen Abgabe von 5,6-Benzo-alpha-pyron zu schaffen, das die vorstehend beschriebenen Nachteile nicht aufweist.

Diese Aufgabe wird erfindungsgemäss durch ein transdermales therapeutisches System (TDS) mit den Merkmalen des Patentanspruchs 1 gelöst. Das TDS enthält den Wirkstoff Benzopyron in einer galenischen Formulierung, die Schweiss aufnehmen kann und durch den Einfluss von Schweisswasser eine konstante Freisetzung des Wirkstoffes während mindestens 24 Stunden ermöglicht. Besonders vorteilhafte Ausgestaltungen der Erfindung und ihrer Verwendung gehen aus den abhängigen Ansprüchen hervor. Die Formulierung kann in einer teilweise aus einer mikroporösen, den Wirkstoffaustritt steuernden Membran gebildeten Hülle eingeschlossen sein.

Die treibende Kraft für die Wanderung des Wirkstoffs bildet der eindringende Schweiss, der die konzentrierte galenische Formulierung des in Wasser und Schweiss wenig löslichen Wirkstoffs ständig am Rande der Uebersättigung hält. Die Kontrolle der gleichmässigen Schweissaufnahme geschieht dabei durch die Formulierung selbst, so dass eine die Wasseraufnahme steuernde Membran, wie sie in EP-A-0 391 172 beschrieben wird, unnötig ist.

Entzündungen, sowie von Hauterkrankungen oder hautnahen Erkrankungen oder zur Behandlung von Hautkrebs im Früh- oder im postoperativen Stadium.

Ein weiterer Grund, der die pharmazeutische Industrie bis jetzt von der Entwicklung eines für humantherapeutische Zwecke geeigneten TDS zur Abgabe von Benzopyron abgehalten hat, ist die Annahme, dass Benzopyron nur ein Prodrug, dagegen sein Hauptmetabolit, nämlich das 7-Hydroxybenzopyron, der - eigentliche Wirkstoff ist (vgl. Sharifi et al., J. Irish Coll. Physicians Surgeons 22, 29-32, 1993).

Bei einer oralen Applikation unterliegt Benzopyron (I) in der Leber einem nahezu quantitativen "first pass"-Metabolismus, d.h., es wird in der Leber sofort und nahezu quantitativ metabolisiert. Das Benzopyron (I) wird dabei irreversibel zum 7-Hydroxy-benzopyron (II) oxidiert, das in einer enzymatisch gekoppelten Reaktion sofort zum pharmakologisch unwirksamen entsprechenden Glukuronid (III) weiterreagiert. Diese Reaktionen laufen sehr schnell ab, wobei der zweite Schritt dieser gekoppelten metabolischen Umwandlung noch ca. 3.5 x schneller als die vorausgehende Oxidationsreaktion erfolgt, so dass praktisch nur sehr geringe Mengen des 7-Hydroxybenzopyrons (II) und praktisch kein unverändertes Benzopyron (I) aus der Leber in den Kreislauf gelangt. Benzopyron kann demzufolge nach oraler Verabreichung das mögliche Zielgewebe im Körper nicht unverändert erreichen.

Die Fachwelt vermutet jedoch, dass ausserhalb der Leber eine weitere Gleichgewichtsreaktion abläuft, bei der ein Teil des in der Leber gebildeten Glukuronids (III) noch vor der an sich rasch erfolgenden Ausscheidung in der Niere wieder zu (II) hydrolysiert wird. Mit anderen Worten, man nimmt an, es werde eine gewisse Menge des 7-Hydroxy-benzopyrons wieder aus dem Glukuronid freigesetzt (vgl.Casley-Smith und Casley-Smith in: High Protein Oedemas and Benzopyrones, Kapitel 9, Seite 423, Lippincott Co., Sydney, 1986).

Die Fachwelt sieht aus verschiedenen Gründen heute das 7-Hydroxybenzopyron als das eigentlich wirksame Agens an. Sharifi et al.,1993 (vorstehend zitiert) geben z.B. auf Seite 29 der vorgenannten Publikation den Stand der Technik wie folgt an: "It is concluded, that coumarin must be a prodrug and that the observed pharmacological effects result most likely from 7-hydroxycoumarin showing measurable concentrations in the systemic circulation."

Da nach-derzeitiger Fachmeinung das 7-Hydroxybenzopyron der eigentliche Wirkstoff ist und somit möglichst hohe Blutspiegel von 7-Hydroxybenzopyron (II) bzw. (unter Berücksichtigung des vorstehend genannten hydrolytischen Gleichgewichts) des entsprechenden Glukuronids (III) anzustreben sind, ist nach Meinung der Fachleute die orale Anwendung der dermalen Applikation überlegen, da erstere zu wesentlich höheren Blutkonzentrationen von 7-Hydroxy-benzopyron führt als die dermalen Applikationsformen.

Dass die orale Applikation tatsächlich zu wesentlich höheren Blutspiegeln an 7-Hydroxybenzopyron führt als die transdermale Applikation, wird auch durch die in Tabelle 3 angeführten klinischen Versuche bestätigt.

Aus der Publikation von Sharifi et al. (1993) geht auch hervor, dass der normalerweise sehr geringe Anteil von II neben viel III im Plasma (Verhältnis 1:100) sich durch sehr hohe orale Dosen von Benzopyron (z.B. 2 g per os) bis auf 1:10 steigern lässt. Dieser, durch akute Absättigung der Kapazität der Glukuronisierungsreaktion in der Leber erklärbare Befund führt dazu, dass man in neueren Therapieversuchen die oralen Benzopyron-Dosen immer weiter steigert (bis zu 7 g/Tag), in der Hoffnung, damit auch den Anteil des allein als wirksam angesehenen 7-Hydroxybenzopyrons (II) im Blut weiter zu erhöhen.

Gemäss der Publikation von Sharifi et al. (1993) bleiben die Blutspiegel von Benzopyron (I) jedoch auch bei sehr hohen oralen Dosen von I sehr gering, da die Fähigkeit der Leber zur Oxidation von Benzopyron, im Gegensatz zur Glukuronisierung, auch bei Applikation sehr hoher Dosen nicht durch Substratmangel begrenzt ist. Die auch in der therapeutischen Praxis übliche Bevorzugung der oralen Applikation von Benzopyron beruht demnach auf der als gesichert geltenden Annahme, dass Benzopyron erst über die nachgewiesene rasche und vollständige Metabolisierung in der Leber wirksam wird. Es sind möglicherweise diese letztgenannten Gründe, die die entsprechend informierten Fachleute trotz gewissen orientierenden Vorversuchen (vgl. Ritschel und Barkhaus, Arzneimittelforschung 38, 1774-1777, 1988) bisher von der auch technisch sehr aufwendigen Entwicklung eines für humantherapeutische Zwecke verwendbaren TDS mit Benzopyron abgehalten haben, da diese Entwicklung ihnen im Vergleich zur oralen Applikationsform von vornherein als wenig sinnvoll erscheinen musste. Dieses nach vorstehenden Ausführungen verständliche Vorurteil kommt beispielsweise in der jüngsten pharmakokinetischen Untersuchung zum Ausdruck, worin der Schluss gezogen wird, dass es unwahrscheinlich sei, dass Benzopyron selbst signifikante pharmakologische Effekte in der systemischen Zirkulation hervorruft (Sharifi et al.,1993, dort Seite 32):" ...coumarin itself is unlikely to produce significant pharmacological effects in the systemic circulation."

Es bestand also ein sehr starkes Vorurteil gegen die Entwicklung eines TDS mit Benzopyron, obwohl von der Hautpenetration bei Ratten her eine solche Möglichkeit grundsätzlich erwogen wurde (Ritschel und Hussain, Meth. Find. Exptl. Clin. Pharmacol.10, Seite 168, 1988, sowie Ritschel und Barkhaus, 1988, wie vorstehend zitiert).

Insgesamt blieben alle diese Versuche auf Untersuchungen an matrix-gesteuerten TDS beschränkt, so auch der in der deutschen Patentschrift 3 715 990 (Beispiel 7) beschriebene Versuch der Polymerisation einer Cumarin enthaltenden Mischung zur Herstellung einer matrixgesteuerten Form eines transdermalen Systems. Die Angaben in dieser deutschen Patentschrift enthalten jedoch keine Details zur technischen und humantherapeutischen Realisation der Erfindung. Insbesondere fehlt jeder Anhaltspunkt dafür, ob das aus der Polymerisationsmischung hergestellte Benzopyron-haltige Polymerisat überhaupt Benzopyron freisetzen kann und ob und wie die eventuell mögliche Freisetzung zeitlich abläuft, insbesondere, ob sie gleichmässig erfolgt. Ueber diese für jedes neue TDS erforderliche Mindestanforderung enthält die genannte Patentschrift keine Informationen.

-Keine der vorgenannten Arbeiten und Publikationen zum Stande der Technik legt demzufolge nahe, wie sich die bekanntermassen schwierige technische Entwicklung eines praktisch, d.h. humantherapeutisch brauchbaren TDS auf der Basis von Benzpyron gestalten sollte.

Aus der Tatsache, dass trotz der seit langem bekannten Feststellung, dass Benzopyron die Haut zu penetrieren vermag, keine konkreteren Angaben, weder durch Publikationen noch in Patentschriften, zur Entwicklung eines humantherapeutisch anwendbaren TDS gemacht wurden, ist zu schliessen, dass die Realisierung eines solchen Vorhabens aus technischen Gründen oder wegen der dargelegten Vorurteile aufgegeben wurde. Der bisher bekannte Stand der Technik lässt die erfindungsgemässe Teilaufgabe, ein humantherapeutisch anwendbares TDS auf der Basis des Wirkstoffs Benzopyron zu entwickeln, als noch ungelöst erscheinen.

Unter Ueberwindung des geschilderten Vorurteils, dass zur Erzielung therapeutischer Effekte möglichst hohe Blutspiegel des 7-Hydroxymetaboliten von Benzopyron anzustreben seien, da dieses das wirksame Agens sei, wurde nun überraschend festgestellt, dass sich das Benzopyron bei Applikation in Form des erfindungsgemässen TDS bevorzugt in entzündeten Zielgeweben anreichert und dort in konzentrierter Form seine pharmakologischen Wirkungen entfaltet, wobei eine metabolische Umwandlung des im Zielgewebe, also lokal, hoch konzentrierten Benzopyrons in 7-Hydroxybenzopyron als Wirkungsmechanismus nicht ausgeschlossen wird.

Darüber hinaus hat dieses Ergebnis der erfindungsgemässen transdermalen Applikation des Benzopyrons als TDS, bei welcher dieses unter Umgehung der vorzeitigen Metabolisierung in der Leber sehr hohe Blut- und Gewebespiegel an unverändertem Benzopyron ergibt, einen weiteren erheblichen Vorteil:

Gegenüber der heute generell üblichen peroralen Verabreichung kann sich Benzopyron bei Applikation als TDS (trotz der relativ geringeren absorbierten Dosis) in verhältnismässig hoher Konzentration im Zielgewebe anreichern, ohne den übrigen Körper toxikologisch unnötig zu belasten. Damit ist die erfindungsgemässe Anwendung von Benzopyron als TDS der üblichen oralen Therapie überlegen.

Im Rahmen der Entwicklung eines TDS, das Benzopyron als Wirkstoff in einer konzentrierten Lösung oder Mikroemulsion statt in einer Polymermatrix enthält, mussten an die Eigenschaften der in dem zu entwickelnden TDS zu verwendenden Formulierungen bzw. an das TDS als Ganzes die folgenden speziellen technisch-physikalischen Anforderungen gestellt werden:
- Die Konzentration des Benzopyrons in der galenischen Formulierung muss möglichst hoch sein, um bei Anwendung des TDS einen ausreichend hohen Konzentrationsgradienten während mindestens 24 Stunden zu gewährleisten.
- Die pro Zeiteinheit abgegebene Dosis muss gleichmässig und hoch genug sein, um den gewünschten therapeutischen Zweck zu erfüllen.
- Schweisswasser aus der Haut, das sich unter einem Okklusionsverschluss immer kondensiert, bildet ein grosses Problem. Es musste eine Formulierung gefunden werden, die das immer entstehende Schweisswasser aufnimmt, ohne sich dabei irreversibel zu verändern, d.h., die Lösung oder die Mikroemulsion darf sich bei der Anwendung auf der Haut nicht entmischen.
- Das TDS muss bei üblichen Lagerungsbedingungen ausreichend lagerstabil und temperaturresistent sein. Die Problematik dieser Stabilitätsanforderung wurde z.B. im EP-A-0 391 172 eingehend diskutiert.
- Das TDS muss ausreichend lange, fest und zuverlässig auf geeigneten Hautstellen haften.
- Schliesslich darf das TDS keine allergisierenden oder hautreizenden Nebenwirkungen besitzen.

Die nachstehenden Ausführungen zeigen nun, dass die vorstehend genannten sehr hohen Anforderungen durch das erfindungsgemässe Arzneimittel erfüllbar sind.

Für die technisch-galenische Entwicklung eines TDS mit einer Benzopyron enthaltenden Formulierung kann die Steuerung der Abgabe des Wirkstoffs aus der Benzopyron-haltigen Formulierung im Innern des TDS auf die Hautoberfläche in an sich bekannter Weise mittels einer üblichen mikroporösen Membran erfolgen. Diese mikroporöse Membran besteht in der Regel aus einem für den Wirkstoff undurchlässigem Material und weist eine vorbestimmte Anzahl mikroskopisch kleiner Perforationen mit festgelegtem Durchmesser auf. Solche Membranen sind dem Fachmann bekannt und auch käuflich erhältlich. Neben der festzulegenden Dimensionierung und den Eigenschaften der Membran ist die Zusammensetzung und Stabilität der Formulierung des Wirkstoffs im Innern des TDS für die praktische Brauchbarkeit eines solchen TDS von entscheidender Bedeutung. Als Formulierungen kommen viskose Lösungen, Gele, Emulsionen oder Mikroemulsionen in Frage, die eine möglichst hohe Konzentration an Wirkstoff enthalten müssen, um das Volumen der Formulierung im Wirkstoffreservoir des TDS möglichst klein und gleichzeitig die Menge des verfügbaren Wirkstoffs möglichst gross halten zu können. Wie bereits angeführt, müssen die Formulierungen bestimmte Mindestanforderungen an die Lagerungsstabilität, sowie gegen eine Entmischung unter Aenderung der Freigabekinetik bei Zutritt von Schweisswasser erfüllen.

Eine solche Formulierung für ein TDS muss bis zu 20% Wasser (als Schweiss) aufnehmen können, das bei Applikation des TDS infolge inverser Osmose durch die Haut in die Formulierung übertritt. Wäre eine solche Formulierung nicht aufnahmefähig für Wasser, so würde die Mikroemulsion im Innern des Pflasters gebrochen bzw. die Lösung oder das Gel entmischt werden, wodurch die Charakteristik der Wirkstoffabgabe verändert und damit das TDS ausser Funktion gesetzt würde. In den Beispielen 1-9 sind solche TDS beschrieben.

Bei der Entwicklung eines TDS musste das Problem gelöst werden, die Beständigkeit und Funktionsfähigkeit der Formulierung aufrechtzuerhalten, trotz des, vor allem bei längerer Applikationsdauer eines TDS zu erwartenden Eindringens erheblicher Mengen von Schweiss.

Es wurden Formulierungen mit 5-50%, vorzugsweise 10-30% Benzopyron ausgewählt, die als Basiskomponenten wechselnde Mengen von emulgierend wirkenden Polymerverbindungen und gegebenenfalls zusätzlich weitere Formulierungsbestandteile wie Konsistenzfaktoren, Emulgatoren, Lösungsvermittler sowie Lösungsmittel mit penetrationssteigernden Eigenschaften enthalten. Solche Verbindungen sind nach dem Stande der Technik bekannt und käuflich erhältlich.

Als emulgierend wirkende Polymere eignen sich hydrophile, hydrophobe und vorzugsweise wasserlösliche Polymere unterschiedlicher Kettenlänge, vorzugsweise aus der Gruppe der Polyalkylenglykole, z.B. entsprechende Polyethylenglykole.

Als Konsistenzfaktoren eignen sich z.B. höhere Fettsäurester von Polyolen, z.B. des Glycerins. Konsistenzfaktoren enthalten z.B. entsprechende Mono- oder Diglyceride, z.B. der Palmitinoder Stearinsäure, oder Gemische solcher Mono- oder Diglyceride, die gemäss CTFA-Nomenklatur als "Glycerylstearate" bezeichnet werden. Als Beispiel für ein spezielles Handelsprodukt dieser Art wird Cutina MD genannt.

Als weitere Emulgatoren und/oder Lösungsvermittler eignen sich anionische, kationische und vorzugsweise nicht-ionogene ("non-ionic") Emulgatoren. Als nicht-ionogene Emulgatoren eignen sich vorzugsweise höhere Fettalkohole mit 8-24, vorzugsweise 16-18 C-Atomen, wie Cetylalkohol und Stearylalkohol, oder Polyglykolester höherer Fettsäuren.

Spezielle Lösungsvermittler enthalten Verbindungen mit grossen hydrophoben und hydrophilen Gruppen, z.B. polyethoxylierte Fettsäureglyceride oder Fettsäurepolyalkylenglykolester, wie z.B. der Rizinusölfettsäuren. Spezielle Beispiele für solche Produkte sind z.B. Cremophore, wie Cremophor RH 40.

Als Lösungsmittel mit penetrationsfördernden Eigenschaften eignen sich nach dem Stande der Technik bekannte hautverträgliche "penetration enhancers" geringer Toxizität, insbesondere niedermolekulare, polare Verbindungen oder deren Gemische, beispielsweise Dimethylsulfoxid (DMSO), Dimethylformamid (DMF) und/oder N-Methylpyrrolidon.

Für die hautabgewandte, als Abdeckfolie ausgebildete Schutzschicht der TDS ("backing") können übliche hautfarbene, z.B. beidseitig mit Polyethylen beschichtete Aluminiumfolien verwendet werden.

Als Membranen zur Steuerung der Diffusion des Wirkstoffs werden handelsübliche mikroporöse Polymermembranen verwendet, die bereits vom Hersteller mit einer Haftschicht (meist Acrylat-oder Silikonkleber) und mit einer nach Herstellung des TDS, vor dem Aufbringen auf die Haut abziehbaren Schutzfolie ausgerüstet sind. Diese Membranen sind aus einem direkt mit der Abdeckfolie verschmelzbaren Material gefertigt, z.B. aus Polyethylen. Es sind auch entsprechende Membranen aus Cellulose, Celluloseacetat, Cellulosenitrat, Polyamid oder Polysulfon bekannt.
Als Beispiele für bevorzugte Steuermembranen werden genannt: Cotran MSP 101088 (MSXE 61), Cotran 61588 (MSXE 62) sowie Cotran 9711 (MSX 1137) der 3M Company, deren Durchlässigkeit für Luft als Mass für die Steuereigenschaften dient. Brauchbare Steuermembranen haben beispielsweise eine Luftdurchlässigkeit von 50 ml in 5-150, vorzugsweise 20-85 Sekunden, je nach dem Grad der gewünschten Steuerfunktion. Die absolut auf der Hautoberfläche verfügbare Menge Benzopyron hängt ausser von der Art und Porenweite der Steuermembran vor allem auch von ihrer aktiven (nutzbaren) Fläche im TDS ab. Diese kann bis zu 300 cm², vorzugsweise 1-100 cm² betragen. Die Schichtdicke der Formulierungen in einem TDS beträgt 0,1-10,0 mm, vorzugsweise 0,1-3,5-mm. Die ausgewählte Schichtdicke hängt vor allem von der vorgesehenen Dauer der Applikation eines TDS ab.

Als die besten, gegen Schweisswasser resistenten Formulierungen erwiesen sich solche mit Polyalkylenglykolen, besonders Polyethylenglykolen, insbesondere PEG 400, ggf. im Gemisch mit höhermolekularen Polyethylenglykolen, wie PEG 1540 oder PEG 4000, Cremophoren, besonders Cremophor RH 40, ggf. unter Zusatz weiterer hautverträglicher Emulgatoren, wie Mono- und/oder Diglyceride von Fettsäuren oder höherer Fettalkohole mit 8-24, vorzugsweise 16-18 C-Atomen, wie Cetylalkohol, sowie üblicher penetrationsfördernder Lösungsmittel wie Dimethylformamid, Dimethylsulfoxid und/oder N-Methylpyrrolidon.

Die verschiedenen Versuchsformulierungen wurden im Wasseraufnahmetest auf ihre Stabilität gegen Schweisswasser getestet. Dazu wurden je 100 ml Formulierung unter Rühren langsam mit 50 ml Wasser oder künstlichem Schweiss aus einer Bürette versetzt, bis die Formulierung bzw. Mikroemulsion einen sprunghaften Anstieg der Tyndall-Streuung bei 610 nm ergab, der auf eine Umwandlung der Formulierung (z.B. Mikroemulsion) unter Entmischung in eine makromycelläre Struktur schliessen lässt.

Die besten Formulierungen können mindestens 20 % Wasser oder Schweiss aufnehmen, ohne dass die Lösungen oder die mikromycellären Strukturen der Mikroemulsionen sich entmischen oder sich erkennbar verändern.

Je 10 ml der aus diesen Versuchen erfolgreich hervorgehenden Formulierungen wurden in einem Test in einem geschlossenen Rohr 14 Tage lang tagsüber abwechselnd entweder 8 h auf +70°C erhitzt oder auf unter 0°C abgekühlt und zwischendurch (über Nacht) jeweils 16 h lang bei Raumtemperatur gehalten. Die besten Formulierungen bestanden auch einen derartigen mehrtägigen Stabilitätstest ohne erkennbare Veränderungen. Diese wurden dann für die Herstellung der unten genannten TDS verwendet und diese zusätzlichen in vitro Diffusions- und Wirkstoffabgabetests unterworfen, ehe sie im klinischen Versuch erprobt wurden.

In den nachstehenden Beispielen 1-9 werden ausgewählte Ausführungsformen für die erfindungsgemässen TDS beschrieben. Die dort aufgeführten Formulierungen bilden aber keine abschliessende Aufzählung der erfindungsgemässen Möglichkeiten. Die verwendeten Rohstoffe und Materialien sind in publizierten Patentschriften und in publizierten Uebersichtsarbeiten ausreichend und ausführlich beschrieben, dem Fachmann bekannt und leicht zugänglich, so dass an dieser Stelle nicht im Detail auf diese an sich bekannten Grundlagen des Standes der Technik hingewiesen werden muss. Dem Fachmann ist auch bekannt, dass man einzelne der genannten Formulierungsbestandteile ohne erfinderisches Dazutun durch funktionell gleichwertige andere handelsübliche Formulierungsbestandteile ersetzen kann, ohne den Rahmen dieser Erfindung zu verlassen.

### Beispiel 1

120 g Benzopyron, 240 g Cremophor RH 40 und 240 g Polyethylenglykol 400 werden bei 65°C gemischt, 30 min bei der gleichen Temperatur gerührt und je ein Gramm der homogenen, klaren Mischung in eine vorbereitete, auf drei Seiten bereits mit einer hautfarbenen Polyethylenfolie verschweisste, mit Klebeschicht und Schutzfolie versehene Steuerfolie blasenfrei abgefüllt und dann durch Verschweissen verschlossen.

### Beispiel 2

150 g Benzopyron, 400 g Cremophor RH 40, 420 g Polyethylenglykol 400, 20 g Cutina MD uns 10 g Cetylalkohol werden bei 65°C gemischt, 30 min gerührt und 1.3 g der Mischung wie in Beispiel 1 abgefüllt.

### Beispiel 3

20 g Benzopyron und 80 g Polyethylenglykol 400 werden bei 60°C vermischt, durch Rühren bei dieser Temperatur gelöst und dann unter Rühren innerhalb einer Stunde abgekühlt. Die klare Mischung kann, wie unter Beispiel 1 beschrieben, abgefüllt werden.

### Beispiel 4

200 g Benzopyron, 790 g Polyethylenglykol 400 und 10 g Cremophor RH 40 werden bei 65°C gemischt und durch halbstündiges Rühren gelöst und wie in Beipiel 1 beschrieben, abgefüllt.

### Beispiel 5

100 g Benzopyron, 200 g Cremophor RH 40, 130 g Polyethylenglykol 1540, 510 g Polyethylenglykol 400, 30 g Polyethylenglykol 4000, 10 g Cetylalkohol und 20 g Cutina MD werden bei 60°C gemischt und innerhalb einer Stunde auf Raumtemperatur abgekühlt. Die entstandene Emulsion wird, wie in Beispiel 1 angegeben, abgefüllt.

### Beispiel 6

60 g Benzopyron, 120 g Cremophor RH 40, 190 g Polyethylenglykol 400, 12 g Polyethylenglykol 4000, 12 g Cutina MD und 6 g Cetylalkohol werden bei 60°C vermischt und eine Stunde lang unter Rühren auf Raumtemperatur abgekühlt. Die viskose Mischung wird, wie unter Beispiel 1 angegeben, abgefüllt.

### Beispiel 7

40 g Benzopyron, 100 g Polyethylenglykol 400, 40 g Dimethylsulfoxid und 20 g Cremophor RH 40 werden bei Raumtemperatur vermischt und nach 30 min Rühren wird die klare Mischung, wie in Beispiel 1 angegeben, abgefüllt.

### Beispiel 8

200 g Benzopyron, 400 g Polyethylenglykol 400, 150 g Dimethylsulfoxid und 150 g N-Methylpyrrolidon werden bei Raumtemperatur gemischt, 30 min gerührt und die klare Mischung, wie in Beispiel 1 angegeben, abgefüllt.

### Beispiel 9

150 g Benzopyron, 300 g Cutina MD und 550 g Polyethylenglykol 400 werden bei 60°C gemischt und langsam, innerhalb einer Stunde, unter Rühren abgekühlt. Die entstandene Mischung ist thixotrop.

Nach dem Wiedererwärmen kann diese Mischung, wie in Beispiel 1 angegeben, abgefüllt werden.

Die Vorauswahl der zu testenden TDS erfolgte durch in vitro-Messungen der Dialyse von Benzopyron aus den untersuchten TDS. Dazu wurden die zur Auswahl stehenden TDS auf der der Haut zugewandten Seite, also membranseitig, mit der Haftschicht in eine Dialyseapparatur geklebt und gegen eine überschüssige Lösung von Polyethylenglycol 400 oder gegen eine Albumin- oder Kochsalzlösung mindestens 24 h lang dialysiert. Zur Messung der diffundierten Menge Benzopyron wurde die Absorption der Dialyselösung spektroskopisch bei 274 nm untersucht. Aus dem linearen Teil der Dialysekurven wurden die Halbwertszeiten für eine 50%ige bzw. 95%ige Diffusionsausbeute (t/2 50% bzw. t/2 95%) berechnet. Beispielsweise ergaben drei ausgewählte Formulierungen die folgenden Halbwertszeiten (Tabelle 1):

**Tabelle 1:**

| **Formulierungs-Nr.** | **Steuermembran Typ MSXE Nr.** | **Halbwertszeit t/2 (h)** |
|---|---|---|
| **1** | **61** | **17** |
| **1** | **62** | **6** |
| **(gemäss Beispiel 5)** | **61** | **60** |
| **(gemäss Beispiel 5)** | **62** | **27** |
| **(gemäss Beispiel 6)** | **61** | **55** |
| **(gemäss Beispiel 6)** | **62** | **21** |

Bei Verwendung einer Cellulosemembran mit einer Porenweite von 20 kD und bei Verwendung einer konstanten aktiven Oberfläche von 2,5 cm² steigt die dialysierbare Menge an Benzopyron aus einer 20%igen Formulierung mit zunehmender Schichtdicke bis zur Erreichung der Sättigungsgrenze der Steuermembran an und bleibt dann trotz weiterer Erhöhung der Schichtdicke konstant (Tab.2):

**Tabelle 2:**

| **Schichtdicke der TDS-Formulierung (mm)** | **Diffundierende Menge Benzopyron im gleichen Zeitraum von 8 h (mg)** |
|---|---|
| **0.1** | **8** |
| **0.2** | **14** |
| **0.4** | **14** |

Die Porenweite der Steuermembran beeinflusst die auf der Hautoberfläche verfügbare Menge Benzopyron erheblich, allerdings steigt sie nicht linear mit der Porengrösse an.
Bei Verwendung der gleichen Formulierung stieg beispielsweise die Diffusionsrate bei Vergrösserung des Porenvolumens von 20 kD auf 100 kD nur um den Faktor 1.6 an.Durch Anstieg der Viskosität der Formulierung wird die Dialysegeschwindigkeit des Benzopyrons stark verringert.

Die Dosis des auf die Hautoberfläche gelangenden Benzopyrons in den erfindungsgemässen membrangesteuerten TDS kann somit über die Variation folgender Parameter beeinflusst werden:
- Basisformulierung ( Zusammensetzung, Konzentration an Benzopyron, Viskosität)
- Schichtdicke der Formulierung im TDS
- Auswahl der Steuermembran unter besonderer Beachtung
   -- der aktiven Oberfläche und
   -- der Porengrösse.

Ueberraschenderweise wurde bei diesen in vitro-Versuchen gefunden, dass die Wirkstoffdiffusion durch die Membran, welche infolge der mit der Zeit abnehmenden Konzentration im Innern des Reservoirs, einer Kinetik erster oder zweiter Ordnung folgen sollte, durch die Aufnahmefähigkeit der bevorzugten Formulierungen für Wasser in vitro nach einer linearen Kinetik erfolgte.

Damit ergibt sich durch das Eindringen von Wasser anstatt der durch die abnehmende Konzentration des Wirkstoffs im Reservoir zu erwartenden nicht-linearen Kinetik, d.h., einer mit der Zeit abnehmenden Wirkstoff-Freisetzung, eine unerwartet gleichmässige (zeitlich konstante) Wirkstoffabgabe durch die Membran. Dieser Befund setzt eine stetig steigende relative Wirkstoff-Freisetzung aus der Wirkstoff-Formulierung im Vergleich zu der im Reservoir verbleibenden Restmenge des Wirkstoffs voraus, die osmotisch durch das Eindringen von Wasser (bzw. Schweiss) bewirkt wird. Dieser überraschende Befund konnte auch im klinischen Versuch mit Probanden, wie nachfolgend beschrieben, bestätigt werden. Dabei wurde, wie nachstehend in der Tabelle 4 gezeigt wird, überraschend gefunden, dass bei einer unerwartet hohen Wirkstoffausnutzung von 70 % während einer 96 stündigen Applikationszeit eines erfindungsgemässen Pflasters konstante Blutspiegel von Benzopyron erzielt werden konnten.

Für die pharmakokinetische Prüfung der erfindungsgemässen TDS am Menschen gab es keine exakten Voraussetzungen.

Insbesondere gab es keine validierte, also ausreichend selektive, empfindliche und genaue analytische Methode, um die in vivo nach oraler und transdermaler Applikation auftretenden Konzentrationen der potentiell wirksamen Komponenten (Benzopyron und 7-Hydroxy-benzopyron) im Blut ausreichend genau messen zu können. Eine vor Beginn der Versuche zu lösende Aufgabe bestand demnach darin, eine solche geeignete analytische Methode zur exakten Kontrolle der transdermalen Bioverfügbarkeit der erfindungsgemässen TDS zu entwickeln. Hierfür gab es nur ungenügende Ansätze: Die Empfindlichkeitsgrenzen der nach dem Stand der Technik bekannten Methoden (Sharifi et al., 1993, dort Fig.2; Egan und O'Kennedy, J. Irish Coll. Physicians Surgeons 22, 72, 1993) lagen z.B. bei 20-36 ng Benzopyron/ml Blutplasma.

Für die beabsichtigten humanpharmakokinetischen Messungen der Absorption von Benzopyron aus einem TDS musste deshalb zunächst eine verbesserte analytische Methode ausgearbeitet werden, bei der die Empfindlichkeit für I und II um den Faktor ca. 50 erhöht war, um auch im erwarteten Grenzkonzentrationsbereich bis 1 ng Benzopyron/ml bzw. 0.1 ng 7-Hydroxybenzopyron/ml noch sichere Aussagen machen zu können.

Dies gelang durch Ausarbeitung einer neuen, hoch sensitiven analytischen Methode.

Zur besseren Vergleichbarkeit wurden manche pharmakokinetischen Vorversuche an ein und demselben männlichen Probanden (also intra-individuell) durchgeführt. Es wurde beispielsweise ein erfindungsgemässes TDS mit 200 mg Benzopyron und einer aktiven Fläche von 12 cm² getestet.

Zum Vergleich wurde dem Probanden in einem weiteren Versuch alle 12 h per os eine Filmtablette (Dragée) mit 100 mg Benzopyron über einen Gesamtzeitraum von 6 Tagen verabreicht, was der gleichen Nominaldosis wie im TDS (200 mg Benzopyron /24 h) entsprach. Die folgenden Blutspiegel wurden gefunden (Tabelle 3):

**Tabelle 3**

| **Ort der Applikation** | **Benzopyron-Dosis und Dauer der Applikation** | **Benzopyron(I)** | | **7-OH-Benzopyron(II)** | | **Relation I/II** |
|---|---|---|---|---|---|---|
| | | **c max (ng/ml)** | **t max (h)** | **c max (ng/ml)** | **t max (h)** | |
| oral | 2 Tabl./24 h (2 x 100 mg/24 h) (6 Tage) | 2.5 | 3 | 4.2 | 4 | 0.6 |
| Oberarm, | 1 TDS/24 h | | | | | |
| innen | (1x 200 mg/24 h) | 25.0 | 8 | 0.47 | 8 | 53.2 |
| Brusthaut | 2 TDS/48 h | | | | | |
| | (2x 200 mg | 24.2 | 3 | 0.24 | 4 | 100.8 |
| | während 48 h) | 36.4 | 34 | 0.65 | 29 | 56.0 |

Die konstante Abgabe von Benzopyron aus dem TDS über die Haut betrug in diesem Fall 35 mg/24 h.

Die vorstehenden Ergebnisse zeigen, dass beim intraindividuellen Vergleich ein fundamental anderes Verhältnis der Blutspiegel von Benzopyron (I) und 7-Hydroxybenzopyron (II) gefunden wird, je nachdem ob die Anwendung oral oder transdermal erfolgt:

Obwohl die via TDS aufgenommene Benzopyrondosis (35 mg/24 h) im vorliegenden Fall nur 17.5 % der oralen Tagesdosis (per os applizierte Menge: 200 mg/24 h) betrug, waren die Blutspiegel an Benzopyron (I) nach transdermaler Applikation 10 x höher als nach oraler Verabreichung und umgekehrt waren die Blutspiegel an II bei Applikation von Benzopyron als TDS 6-10 x niedriger als nach Verabreichung der oralen Dosis.

Das experimentell gefundene Verhältnis von Benzopyron (I) zu 7-Hydroxybenzopyron (II) im Blut ist bei Applikation von I als TDS also um den Faktor 100-200 grösser als nach seiner oralen Applikation (Tabelle 3). Ein derart fundamental anderes Verhältnis der Blutspiegel von I zu II nach Applikation von Benzopyron als TDS war unerwartet.

Da die Blutspiegel nach oraler Verabreichung im steady state (also bei Absättigung des Blutes) gemessen wurden, und bekannt ist, dass die primäre Bioverfügbarkeit oraler Formen von Benzopyron nahezu 100% beträgt, kann die folgende Aussage über den Unterschied der beiden Applikationsformen gemacht werden: Bezogen auf die gleiche pro Tag absorbierte Dosis an Benzopyron (I) sind die maximalen Blutspiegel von (I) nach Applikation als TDS 57 x höher als nach oraler Applikation. Ebenfalls bezogen auf die gleiche absorbierte Dosis sind die maximalen Blutkonzentrationen an II etwa vergleichbar. Da beim TDS, wie bereits aus der vorstehenden Tabelle 3 erkennbar, auch am 2. Applikationstag noch vergleichbar hohe Blutspiegel an I auftreten, resultiert für das TDS mit 200 mg Benzopyron eine relativ hohe Bioverfügbarkeit von 35%/48 h.

Diese für ein TDS bereits sehr hohe Bioverfügbarkeit liess sich in einem Experiment, bei dem ein TDS 4 Tage lang getragen wurde, weiter auf 70 % in 96 h erhöhen. Dies ist eine ausserordentlich hohe Bioverfügbarkeit für ein TDS, da bekanntlich aus thermodynamischen Gründen immer eine Restmenge des Wirkstoffs im TDS erhalten bleiben muss. Dieser Versuch zeigt auch, dass die Abgabe von Benzopyron über 96h (4 Tage) konstant erfolgt. In einem anderen Experiment wurde an einem anderen erfindungsgemässen TDS geprüft, ob die Abgabe von Benzopyron aus dem TDS wirklich über die gesamte Prüfzeit von 96 h konstant ist und wie schnell die Blutspiegel an Benzopyron nach Entfernen des TDS zurückgehen (Tabelle 4):

**Tabelle 4**

| Applikation eines TDS (200 mg Benzopyron) über 96 Stunden | |
|---|---|
| **Applikationsdauer** | **Blutspiegel von Benzopyron(I);** |
| (h) | (ng/ml) |
| 48 | 5.5 |
| 72 | 6.1 |
| 82 | 8.7 |
| 96 | 6.6 |
| | |

| **(nach Entfernen des TDS)** | |
|---|---|
| (h) | (ng/ml) |
| 3 | 2.4 |
| 6 | 0.8 |

Dieses Ergebnis bestätigt, dass die Blutspiegel von Benzopyron über 4 Tage gleichmässig hoch bleiben und nach Entfernen des TDS sehr rasch absinken.

Die im Vorversuch gefundenen Ergebnisse wurden durch eine GCP-konforme pharmakokinetische Studie an 14 Freiwilligen bestätigt (Tabelle 5):

**Tabelle 5**

| Einmalapplikation eines TDS mit 200 mg Benzopyron an 14 Freiwilligen | | | |
|---|---|---|---|
| Applikationszeit | Benzopyron(I) c max(Mittelwert) | 7-OH-Benzopyron(II) c max(Mittelwert) | I/II |
| (h) | (ng/ml) | (ng/ml) | |
| 3 | 23.4 | 0.40 | 58.5 |
| 24 | 27.3 | 0.48 | 56.9 |
| | | | |

| **(nach Entfernen des TDS)** | | | |
|---|---|---|---|
| (h) | (ng/ml) | (ng/ml) | |
| 4 | 4.96 | 0.13 | --- |

Auch dieser klinische Versuch ergab nach Anwendung eines TDS eine konstante Blutkonzentration an Benzopyron von der 3. bis zur letzten (24.) Stunde der Applikation.

Wie aus Tab. 5 ersichtlich, waren die Benzopyronspiegel im Blut bereits 4 Stunden nach Entfernen des TDS auf weniger als 20% der Blutspiegel während der Applikation gefallen. Auch die Blutspiegel von 7-Hydroxybenzopyron sanken rasch ab.
Der "steady state", d.h. die definitive Höhe des dann konstant bleibenden Blutspiegels an Benzopyron, stellte sich bereits nach 3h ein.

Die hohe Nutzausbeute von 70% in 96 h und die gefundene lineare Wirkstoffabgabe über die gesamte Applikationsdauer von 96 h zeigen, dass auch die mit der Absorption des Schweisswassers verbundenen Probleme gelöst werden konnten. Dabei wurde festgestellt, dass das Schweisswasser von den in den Beispielen beschriebenen Benzopyronformulierungen nicht nur absorbiert wird, sondern die konstante Freisetzung von Benzopyron während einer 24 Stunden wesentlich überschreitenden Zeit ermöglicht.

Als weiteres positives Ergebnis der Untersuchungen ist die ausgezeichnete Verträglichkeit und die gute Haftfähigkeit der in den Beispielen beschriebenen TDS zu nennen.

Auch die Lagerungsstabilität der erfindungsgemässen TDS ist gut.

Damit war die Aufgabe, ein praktikables, für humantherapeutische Zwecke geeignetes TDS zu realisieren, erreicht.

## Patentansprüche

1. Transdermales therapeutisches System ohne Polymermatrix enthaltend eine galenische Formulierung mit einem pharmazeutischen Wirkstoff und einer mikroporösen, den Wirkstoffaustritt steuernden Membran, dadurch gekennzeichnet, dass die galenische Formulierung als thixotrope oder viskose Mischung oder als stabile Emulsion ausgebildet ist, 5 bis 50 Gew.% 5,6-Benzo-alpha-pyron enthält und bis zu 20 % Wasser als Schweiss aufnehmen, ohne dass sie gebrochen oder entmischt wird, und durch den Einfluss von Schweisswasser eine konstante Freisetzung des Wirkstoffes während mindestens 24 Stunden ermöglicht.

2. Transdermales therapeutisches System nach Anspruch 1, gekennzeichnet durch eine auf der mikroporösen Membran hautseitig angebrachten Haftschicht für die Hautoberfläche mit einer die Haftschicht abdeckenden, entfernbaren, undurchlässigen Schutzschicht.

3. Transdermales therapeutisches System nach Anspruch 1 oder 2, dadurch gekennzeichnet, dass die mikroporöse Membran eine Porengrösse von 10 - 100 kD besitzt.

4. Transdermales therapeutisches System nach einem der Ansprüche 1 bis 3, dadurch gekennzeichnet, dass die galenische Formulierung 10 bis 30 Gew.% 5,6-Benzo-alpha-pyron, 1 bis 50 Gew.% eines Lösungsvermittlers und 15 bis 85 Gew.% eines oder mehrerer Polyalkylenglykole, oder gegebenenfalls weitere Emulgatoren, sowie Konsistenzfaktoren, Lösungsmittel und/oder höhere Fettalkohole mit 8 bis 24 Kohlenstoffatomen enthält.

5. Transdermales therapeutisches System nach einem der Ansprüche 1 bis 3, dadurch gekennzeichnet, dass die galenische Formulierung 5,6-Benzo-alpha-pyron und mindestens ein Polyethylenglykol enthält.

6. Transdermales therapeutisches System nach Anspruch 5 dadurch gekennzeichnet, dass die galenische Formulierung 10 bis 30 Gew.% 5,6-Benzo-alpha-pyron und 20 bis 90 Gew.% eines oder mehrerer Polyethylenglykole vom Molekulargewicht 300-5'000 enthält.

7. Transdermales therapeutisches System nach Anspruch 5 oder 6 dadurch gekennzeichnet, dass die galenische Formulierung des weitern mindestens einen der folgenden Zusätze enthält:
- bis zu 50 Gew.% eines Cremophors (Lösungsvermittlers),
- bis zu 35 Gew.% eines Konsistenzfaktors aus der Gruppe der Glycerylstearate,
- bis zu 5 Gew.% eines Fettalkoholes mit 16 bis 18 Kohlenstoffatomen,
- bis zu 30 Gew.% eines oder mehrerer Lösungsmittel aus der Gruppe bestehend aus Dimethylsulfoxid, Dimethylformamid und N-Methylpyrrolidon.

8. Transdermales therapeutisches System nach einem der Ansprüche 1 bis 7, dadurch gekennzeichnet, dass die Schichtdicke der galenischen Formulierung maximal 10 mm, vorzugsweise 0,1 - 3,5 mm beträgt.

9. Transdermales therapeutisches System gemäss einem der Ansprüche 1 bis 8 zur Anwendung in der Behandlung von chronischen Entzündungen.

10. Transdermales therapeutisches System gemäss einem der Ansprüche 1 bis 8 zur Anwendung in der Behandlung von Hauterkrankungen und hautnahen Erkrankungen.

11. Transdermales therapeutisches System gemäss einem der Ansprüche 1 bis 8 zur Anwendung in der Behandlung von Hautkrebs im Frühstadium sowie im postoperativen Stadium.

12. Transdermales therapeutisches System gemäss einem der Ansprüche 1 bis 8 zur Anwendung in der Behandlung von Lymphödemen.

13. Transdermales therapeutisches System gemäss einem der Ansprüche 1 bis 8 zur Anwendung in der Behandlung von chronischen Venenentzündungen.

## Claims

1. A transdermal therapeutic system without polymer matrix containing a pharmaceutical formulation with an active ingredient and a microporous membrane controlling the release of the active ingredient, characterised in that the pharmaceutical formulation is a thixotropic or viscouse mixture or a stable emulsion, contains 5 to 50% by weight of 5,6-benzo-alpha-pyrone and can absorb up to 20% of water as perspiration without breaking or disintegrating and, as a result of the effect of perspiration, permits a constant release of the active ingredient during at least 24 hours.

2. A transdermal therapeutic system according to claim 1, characterised in that an adhesive layer is applied to the microporous membrane on the skin side, for the skin surface, having a removable, impermeable backing which covers the adhesive layer.

3. A transdermal therapeutic system according to claim 1 or 2, characterised in that the microporous membrane has a pore size of 10-100 kD.

4. A transdermal therapeutic system according to any one of the claims 1 to 3, characterised in that the pharmaceutical formulation contains 10 to 30% by weight of 5,6-benzo-alpha-pyrone, 1 to 50% by weight of a solubilizer and 15 to 85% by weight of one or more polyalkylene glycols, or optionally further emulsifiers, and consistency factors, solvents and/or higher fatty alcohols having 8 to 24 carbon atoms.

5. A transdermal therapeutic system according to any one of the claims 1 to 3, characterised in that the pharmaceutical formulation consists of 5,6-benzo-alpha-pyrone and at least one polyethylene glycol.

6. A transdermal therapeutic system according to claim 5, characterised in that the pharmaceutical formulation contains 10 to 30% by weight of 5,6-benzo-alpha-pyrone and 20 to 90% by weight of one or more polyethylene glycols having a molecular weight of 300-5,000.

7. A transdermal therapeutic system according to claim 5 or 6, characterised in that the pharmaceutical formulation furthermore contains at least one of the following additives:
- up to 50% by weight of a Cremophor (solubilizer),
- up to 35% by weight of a consistency factor selected from the group consisting of the glyceryl stearates,
- up to 5% by weight of a fatty alcohol having 16 to 18 carbon atoms and
- up to 30% by weight of one or more solvents selected from the group consisting of dimethyl sulfoxide, dimethylformamide and N-methylpyrrolidone.

8. A transdermal therapeutic system according to any one of the claims 1 to 7, characterised in that the layer thickness of the pharmaceutical formulation is not more than 10 mm, preferably 0,1 - 3,5 mm.

9. A transdermal therapeutic system according to any one of the claims 1 to 8 for use in the treatment of chronic inflammations.

10. A transdermal therapeutic system according to any one of the claims 1 to 8 for use in the treatment of skin diseases and diseases close to the skin.

11. A transdermal therapeutic system according to any one of the claims 1 to 8 for use in the treatment of skin cancers in the early stage and in the postoperative stage.

12. A transdermal therapeutic system according to any one of the claims 1 to 8 for use in the treatment of lymphedemas.

13. A transdermal therapeutic system according to any one of the claims 1 to 8 for use in the treatment of chronic phlebitis.

## Revendications

1. Système thérapeutique transdermique sans matrice polymère contenant une formulation galénique avec une substance active pharmaceutique et une membrane microporeuse contrôlant la sortie de la substance, caractérisé en ce que la formulation galénique est conçue en tant que mélange thixotrope ou visqueux, ou en tant qu'émulsion stable, contient 5 à 50 % en masse de 5,6-benzo-alpha-pyrone et qu'elle doit pouvoir incorporer jusqu'à 20 % d'eau en tant que sueur, sans qu'elle ne soit désémulsionnée ou séparée, et qu'elle permette, par l'influence de l'eau de sueur, une libération constante de la substance active pendant au moins 24 heures.

2. Système thérapeutique transdermique selon la revendication 1, caractérisé par une couche d'adhérence, appliquée côté peau sur la membrane microporeuse, pour la surface de peau et doté d'une couche de protection imperméable amovible et recouvrant la couche d'adhérence.

3. Système thérapeutique transdermique selon la revendication 1 ou 2, caractérisé en ce que la membrane microporeuse possède une taille de pores de 10 à 100 kD.

4. Système thérapeutique transdermique selon l'une des revendications 1 à 3, caractérisé en ce que la formulation galénique comprend 10 à 30 % en masse de 5,6-benzo-alpha-pyrone, 1 à 50 % en masse d'un agent de solubilisation et 15 à 85 % en masse d'un ou de plusieurs polyalkylèneglycols, ou éventuellement d'autres émulsifiants, ainsi que des facteurs de consistance, des solvants et/ou des alcools gras supérieurs comportant 8 à 24 atomes de carbone.

5. Système thérapeutique transdermique selon l'une des revendications 1 à 3, caractérisé en ce que la formulation galénique comprend de la 5,6-benzo-alpha-pyronne et au moins un polyéthylèneglycol.

6. Système thérapeutique transdermique selon la revendication 5, caractérisé en ce que la formulation galénique contient 10 à 30 % en masse de 5,6-benzo-alpha-pyrone et 20 à 90 % en masse d'un ou de plusieurs polyéthylèneglycols présentant une masse moléculaire comprise entre 300 et 5 000.

7. Système thérapeutique transdermique selon la revendication 5 ou 6, caractérisé en ce que la formulation galénique contient en outre au moins un des additifs suivants :
- jusqu'à 50 % en masse d'un crémophore (agent de solubilisation)
- jusqu'à 35 % en masse d'un facteur de consistance issu du groupe des stéarates de glycéryle,
- jusqu'à 5 % en masse d'un alcool gras comportant 16 à 18 atomes de carbone,
- jusqu'à 30 % en masse d'un ou de plusieurs solvants issus du groupe composé du diméthylsulfoxyde, du diméthylformamide et de la N-méthylpyrrolidone.

8. Système thérapeutique transdermique selon l'une des revendications 1 à 7, caractérisé en ce que l'épaisseur de couche de la formulation galénique est au maximum égale à 10 mm, de préférence comprise entre 0,1 et 3,5 mm.

9. Système thérapeutique transdermique selon l'une des revendications 1 à 8 destiné à être utilisé dans le traitement d'inflammations chroniques.

10. Système thérapeutique transdermique selon l'une des revendications 1 à 8 destiné à être utilisé dans le traitement de maladies de peau et de maladies touchant la peau.

11. Système thérapeutique transdermique selon l'une des revendications 1 à 8 destiné à être utilisé dans le traitement de cancers de la peau à un stade précoce et à un stade postopératoire.

12. Système thérapeutique transdermique selon l'une des revendications 1 à 8 destiné à être utilisé dans le traitement d'oedèmes lymphatiques.

13. Système thérapeutique transdermique selon l'une des revendications 1 à 8 destiné à être utilisé dans le traitement d'inflammations veineuses chroniques.
